# EUROPEAN PATENT SPECIFICATION

(11) **EP 1 788 962 B1**
(45) Date of publication and mention of the grant of the patent: **02.05.2012**
(21) Application number: 05782802.2
(22) Date of filing: 24.08.2005
(51) Int. Cl.: A61B 17/70

(54) **Device for securing a spinal rod to the spine**
Vorrichtung zur Fixierung einer Wirbelsäulenstange an der Wirbelsäule
Dispositif pour fixer une tige vertébrale sur la colonne vertébrale

(30) Priority: 24.08.2004 US 925295
(43) Date of publication of application: 30.05.2007
(73) Proprietor: Stryker Spine, 33610 Cestas (FR)
(72) Inventor: DAVID, Jérôme, F-33100 Bordeaux (FR)
(74) Representative: Callon de Lamarck, Jean-Robert
(86) International application number: PCT/EP2005/009235
(87) International publication number: WO 2006/021450

(56) References cited:
- FR-A- 2 844 180

## Description

### BACKGROUND OF THE INVENTION

The present invention relates to bone fixation devices and, in particular, to pedicle fixation assemblies.

The spinal column is a highly complex system of bones and connective tissues that provide support for the body and protects the delicate spinal cord and nerves. The spinal column includes a series of stacked vertebral bodies, each vertebral body including an inner or central portion of relatively weak cancellous bone and an outer portion of relatively strong cortical bone. Situated between each vertebral body is an intervertebral disc that cushions and dampens compressive forces exerted upon the spinal column. A vertebral canal containing the spinal cord and nerves is located behind the vertebral bodies.

A surgical technique commonly referred to as spinal fixation uses surgical implants for fusing together and/or mechanically immobilizing two or more vertebral bodies of the spinal column. Spinal fixation may also be used to alter the alignment of adjacent vertebral bodies relative to one another to change the overall alignment of the spinal column. Such techniques have been used effectively to treat a wide variety of conditions and, in most cases, to relieve pain.

One spinal fixation technique involves immobilizing the spine using orthopedic stabilizing rods, commonly referred to as spinal rods, which run generally parallel to the spine. This technique involves exposing the spine posteriorly and fastening bone screws to the pedicles of vertebral bodies. The pedicle screws are generally placed at least one per vertebra and serve as anchor points for the spine rods. Clamping elements adapted for receiving a spine rod therethrough are then used to join the spine rods to the pedicle screws. The aligning influence of the spine rods forces the spinal column to conform to a more desirable shape. In certain instances, the spine rods may be bent to achieve the desired curvature of the spinal column.

Most existing rod fixation systems require several components to build the systems. Each additional component or instrument required to assemble the fixation system adds to the complexity of the surgical technique. A need has thus arisen for improved fixation systems that minimize the assembly of small pieces of hardware during the surgical procedure. Thus, there remains a need for spinal fixation devices that facilitate simple and fast assembly of attachment of a spinal rod to a spine. It would be desirable to provide a device with pre-assembled components that will result in less time being required to assemble the components in the operating room.

Additionally, many of the known existing rod fixation systems require at least a two-step locking procedure which not only lengthens the surgical procedure but also increases the complexity of the procedure. Although some existing rod fixation systems do offer one-step locking such as the spinal fixation system disclosed in U.S. Patent No. 5,534,002, it would still be desirable to have a one-step-locking device with pre-assembled components and/or a one-step-locking device with increased friction surface area between assembled components. A further rod fixation system is disclosed in FR 2 844 180 A1.

### SUMMARY OF THE INVENTION

The present invention is directed towards a device for securing a spinal rod to a spine according to claim 1. The assembly includes an elongated spinal rod, a bone fixation element, a ball ring, a set screw and a rod connector. The elongated spinal rod is configured to be implanted adjacent to a spinal column of a patient and span across vertebral bodies. The bone fixation element of the assembly includes a bone engaging portion, as well as a stem portion. The ball ring of the present invention has an aperture that extends through the center of the ball ring and can slidably receive the spinal rod. Furthermore, the ball ring also includes a through slot that allows the ball ring to be compressed and expanded about a central axis extending through the center of the ball ring. The rod connector of the present assembly is adapted for connecting the bone fixation elements to the spinal rod. The connector includes a body having a channel for housing the ball ring, a stem bore adapted to receive the stem portion of the bone fixation element and a set screw bore having threads which are engagable with the threads of the set screw. The channel is able to communicate with the set screw bore as well as the stem bore.

In certain preferred embodiments, the spinal rod is generally cylindrical. Additionally, the stem portion of the bone fixation element may be smooth.

In other preferred embodiments, the ball ring is captured within the channel of the connector, and the through slot of the ball ring permits the outer diameter of the ball ring to be reduced less than a minimum diameter of the channel. Furthermore, the ball ring may have a relaxed diameter smaller than the diameter of the channel of the rod connector, so as to permit polyaxial motion of the rod when the rod is housed within the aperture of the ball ring.

In some preferred embodiments, the set screw bore may be offset from the stem bore at an angle greater than or equal to 3 degrees with reference taken from a stem bore axis.

Since the channel intersects the stem bore, the ball ring, once inside the channel, is able to apply pressure against the stem of the fixation element when the stem is housed within the stem bore. In order to achieve greater lateral movement of the ball ring within the channel, the channel and the stem bore may have a non-circular geometric configuration, and preferably are elliptical. This non-circular configuration of the channel and stem bore create a situation for the device where, in a locked position, the ball ring may either apply pressure against or receive pressure from at least three contact points, while the stem may also apply pressure against or receive pressure from at least three contact points. The stem bore may also be offset at an angle between 3 degrees and 15 degrees relative to a second axis extending through the maximum distance of the channel. Furthermore, the set screw bore may be aligned with this second axis.

In an alternate embodiment, the spinal rod assembly may further include a second ball ring. With the addition of the second ball ring, the connector may be constructed similar to the previous embodiment described, with the exception that the second ball ring is now positioned about the stem of the bone fixation element. Thus, instead of the first ball ring contacting the stem when it is moved in a lateral direction, the first ball ring applies pressure against the second ball ring. This causes the second ball ring to tighten around the stem while the first ball ring tightens around the rod as pressure is applied to the first ball ring by the set screw.

In an alternate embodiment, the set screw may have a configuration which prevents it from being backed all the way out of the set screw bore. Consistent with this approach, the set screw may have to be placed within the connector by maneuvering the set screw through the channel and up through the set screw bore.

In a method of use of the present invention, the bone fixation element may first be engaged with a vertebral body. The connector is then placed over the stem portion of the bone fixation element, wherein the channel of the connector is in communication with both the stem bore and the set screw bore. The set screw and the ball ring may be placed within the connector, either before the connector is placed over the stem portion or after the connector is placed over the stem portion. The spinal rod then may be slid through the aperture of the ball ring and maneuvered until a correct position for the rod is achieved. In order to lock the rod and stem relative to one another, the set screw may then be translated downward through the set screw bore until it applies a force against the ball ring. This forces the ball ring to tighten around the rod and move in a lateral direction towards the stem. As the set screw is continually translated downward through the set screw bore, the stem comes in contact with the interior wall of the stem bore until it is locked between the ball ring and the set screw bore. Additionally, the ball ring is further tightened around the rod until the ball ring is locked between the set screw and the stem. At this point, the rod and stem are locked relative to the connector.

The stem bore may have a larger diameter than the stem in order to allow polyaxial motion of the stem within the connector. Additionally, the set screw, as well as the bone fixation element, may have recesses for cooperating with a tool.

The present invention as described herein may also be provided within a kit. The kit may include a plurality of connectors, a plurality of bone fixation elements, a plurality of spinal rods, a plurality of set screws, a plurality of ball rings and any combination thereof. Furthermore, different size elements may be included within the kit, as well as different types of these elements. For instance, with regard to the bone fixation element, the kit may include pedicle screws and/or hook assemblies or other variations thereof.

### BRIEF DESCRIPTION OF THE DRAWINGS

FIG. 1 illustrates one embodiment of the present invention;

FIG. 2 shows a blow-up prospective view of the embodiment shown in FIG. 1;

FIG. 3 is cross-sectional view of two embodiments shown in FIG. 1 connected to a vertebral body;

FIG. 4 is a prospective view of a bone fixation element;

FIG. 5a is a prospective view of an embodiment of a connector used in the spinal assembly;

FIG. 5b is a cross-sectional view of the connector shown in FIG. 5a;

FIG. 6 is a prospective view of a ball ring;

FIG. 7 is a prospective view of a set screw;

FIG. 8a is a cross-sectional view of the embodiment of FIG. 1 shown in an assembled state;

FIG. 8b is a top view of the assembly shown in FIG. 8a; and

FIG. 9 is a cross-sectional view of an additional device.

### DETAILED DESCRIPTION

Before describing several exemplary embodiments of the present invention, it is to be understood that the present invention is not limited to the details of construction set forth in the following description. The present invention is capable of other embodiments and of being practiced or carried out in various ways.

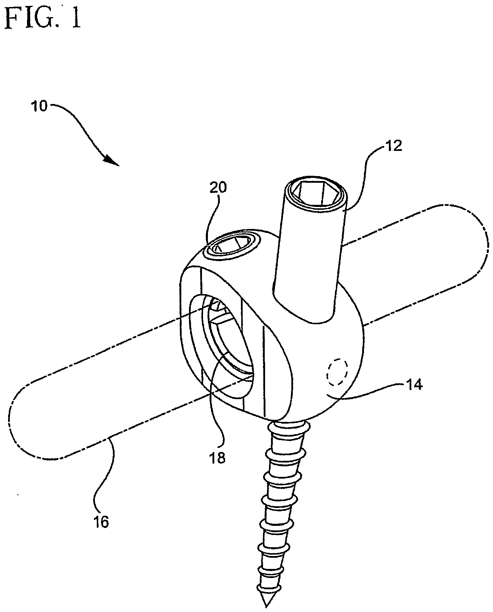

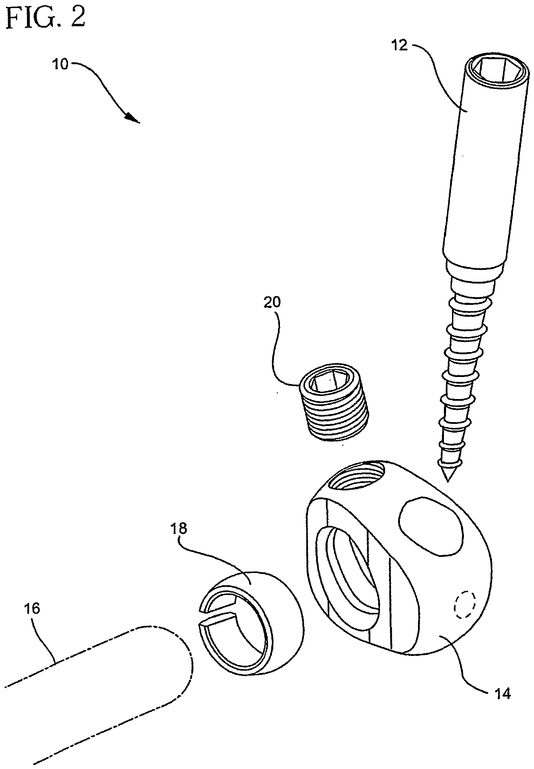

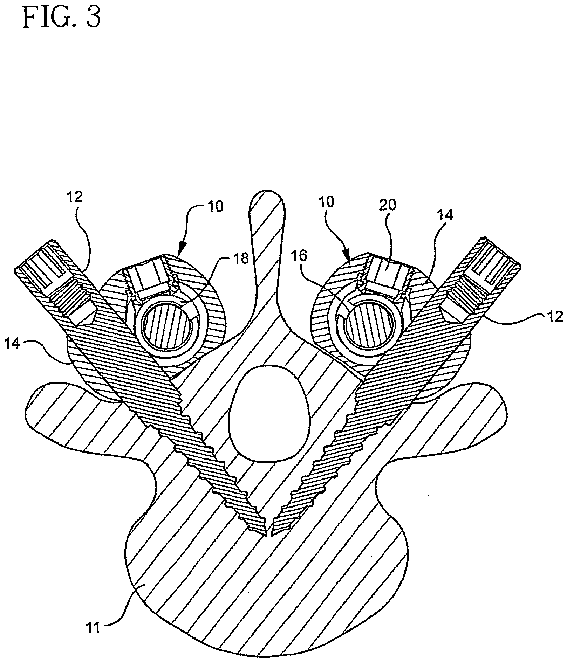

Referring now to the drawings and particularly to FIGS. 1-3, a bone fixation assembly 10, in accordance with certain preferred embodiments of the present invention is shown. The bone fixation assembly may include at least one fixation element 12 such as a bone screw, hook or anchor, a connector 14, an elongated spinal rod 16 which is preferably cylindrical, a ball ring 18, and a set screw 20. The bone fixation assembly 10 may be secured to the pedicles 11 of the vertebral bodies of a spinal column, as shown in FIG. 3.

Connector 14 of bone fixation assembly 10 is preferably made of a biological inert material, for example, any metal customarily used for surgical devices and particularly those used for bone screws and pins, such as titanium or stainless steel. Other suitable materials for the connecting element 14 include, but are not limited to, alloys, composite materials, ceramics, or carbon fiber materials.

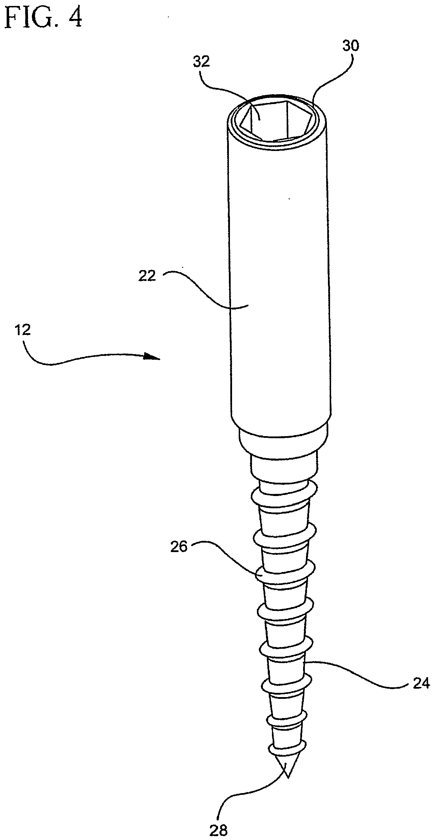

Fixation element 12 as shown in FIG. 4 includes a stem 22 and a bone-engaging portion 24. Stem 22 is preferably cylindrical and preferably has a smooth outer surface to facilitate sliding as will be discussed below. The stem can be constructed in other ways or in connection with other components to facilitate sliding of connector 14 with respect to fixation element 12. Fixation element 12 could be any suitable fixation element for attachment to a bone, for example, a hook or a screw. In preferred embodiments, fixation element 12 comprises a screw or fastener having a stem portion 22 and threaded bone-engaging portion 24. Bone-engaging threads 26 are adapted to be engaged in bone material. Portion 24 of fixation element 12 opposite stem 22 has a tip 28 for insertion into bone, and external screw threads 26 extending between tip 28 and stem 22. Screw threads 26 have an inner root diameter and an outer diameter. Fixation element 12, including screw threads 26 and stem 22, is preferably made of a biological inert material, such as titanium or stainless steel.

In the embodiment shown in FIG. 4, one end of bone fixation element 12 includes a top surface 30, which preferably includes a recess 32. In the embodiment shown, recess 32 is in the form of a hollow hexagonal recess adapted to receive an end of a hexagonal driver for rotating fixation element 12. It should be understood, however, that other internal or external tool engagement recesses can be used according to the present invention.

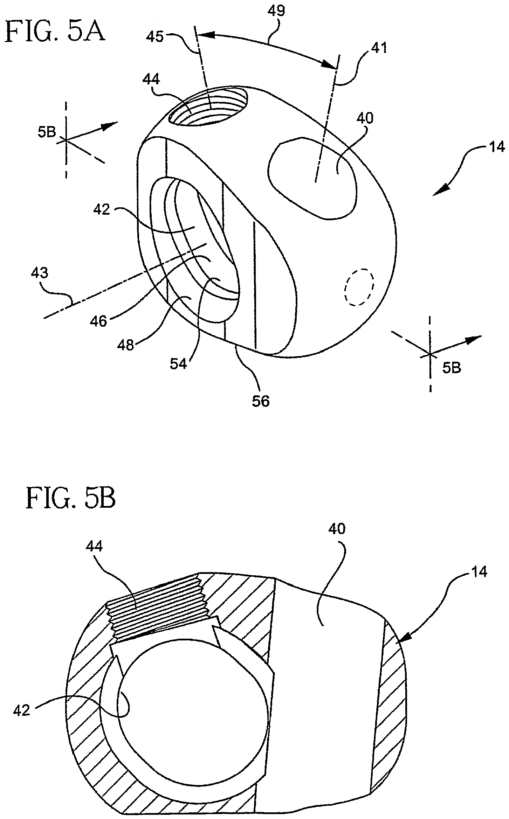

Connector 14, which is shown in more detail in FIGS. 5a and 5b, has at least a first bore 40 having a longitudinal axis 41 extending in the Zenith axis adapted to receive stem 22 of fixation element 12 and to permit axial, sliding movement of connector 14 along the longitudinal axis of stem 22 with respect to fixation element 12. Preferably, first bore 40 has a non-circular shape and most preferably is elliptical. Bore 40 may have a diameter larger than the diameter of stem 22 in order to allow polyaxial motion of fixation element 12 within bore 40. Connector 14 further includes a ball ring receiving bore in the form of channel 42 for receiving and/or housing ball ring 18 which, in turn, receives rod 16. Connector 14 has a set screw bore 44 which intersects channel 42 and extends along axis 45. Channel 42 preferably has a non-circular geometry and may be elliptical in shape. Channel 42 has a longitudinal axis 43 extending in the Y-axis, that is substantially transverse to longitudinal axis 41 of first bore 40. In a preferred embodiment, the angle between axis 45 and axis 41 is in a range between 3 degrees and 15 degrees as shown by arc 49 in FIG. 5a. Axis 41 is preferably at an angle to the plane containing axis 43, extending parallel to the sagittal plane.

In a most preferred embodiment, set screw bore 44 is aligned with channel 42 in the Zenith-axis so as to be offset from bore 40 similarly. It is also within the scope of the present invention that only set screw bore 44 is offset a distance greater than 3 degrees from bore 40 and not channel 42, i.e., the plane containing axis 45 being parallel to the sagittal plane is also parallel to a plane containing axis 41.

Channel 42 may include a recessed portion 46 located within the interior of connector 14 and beveled portion 48 located on both exterior sides 50 and 52 of connector 14. Although recessed portion 46 and beveled portion 48 both may be elliptical in shape, the inner diameter of portion 48 is smaller than the diameter of portion 46. Preferably, a ridge 54 extends between beveled portion 48 and recessed portion 46 on both sides of connector 14. Additionally, beveled portions 48 may have entrances that are tapered inwards reducing the minimum diameter of the beveled portions as you approach recessed portion 46 from the exterior of the connector.

This configuration enables ball ring 18 to be placed within recessed portion 46 by compressing the ball ring diameter and still have a certain degree of motion, because in its relaxed position, the ball ring has a diameter less than the minimum diameter of the recessed portion. However, beveled portion 48 along with ridge 54 prohibits ball ring 18 from being able to withdraw out of recessed portion 46 because the beveled portion has a diameter less than the diameter of ball ring 18 in its relaxed position.

Channel 42 has an opening that intersects with set screw bore 44. This allows a set screw in bore 44 to contact the outer surface of ball ring 18 once the ball ring is housed within recessed portion 46 of channel 42. As shown in FIG. 3, set screw bore 44 includes interior threads and is adapted for receiving set screw 20, which includes mating external threads.

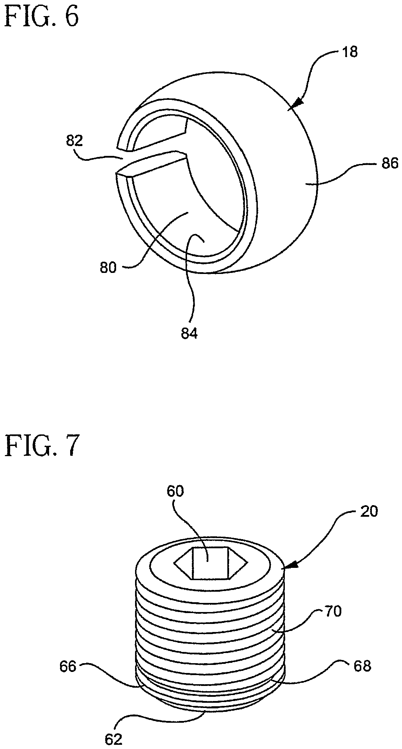

As illustrated in FIG. 6, in the preferred embodiment, ball ring 18 has a generally spherical outer surface having an opening 80 through its body and, on insertion, may be coaxial with channel 42. Ball ring 18 is adapted to receive spinal rod 16, specifically opening 80 of the ball ring receives the rod. As shown in FIG. 6, ball ring 18 includes a through slot 82 extending through the surface of the ball ring. The through-slot 82 allows ball ring 18 to be compressed such that the inner diameter of ball ring opening 80 is reduced when the ball ring is compressed as well as expanded if need be.

As shown in FIG. 7, the threads of set screw 20 are engageable with the threads of set screw bore 44, thus permitting set screw 20 to be threaded downward through set screw bore 44 in order to apply pressure against ball ring 18.

Set screw 20 also includes a recess 60 for engaging a tool. Although the recess is shown in the form of a hexagonal recess adapted to receive an end of a hexagonal driver for turning set screw 20, it will be understood that other internal or external tool engagement features can be used according to the present invention. In a preferred embodiment, the set screw is cylindrical and the surface of set screw 20, which applies pressure against the ball ring, may include a tapered end surface 62. Tapered end 62 contacts ball ring 18 to compress the same as will be described below.

In a preferred embodiment, set screw 20 is sealed or pre-seated within connector 14 such that the set screw cannot be inadvertently removed from the connector. The elimination of any inadvertent removal of set screw 20 can be accomplished by providing a flared portion or a lip 66 on the end of the set screw opposite tool engaging recess 60. The flared portion or lip 66 has a diameter that is greater than the diameter of the threaded portion 70 of set screw 20. Thus, prior to ball ring 18 being placed within channel 42 of connector 14, set screw 20 may be maneuvered through channel 42. Upon reaching the intersection point between channel 42 and set screw bore 44, set screw 20 with the surface containing recess 60 proceeding first, is placed through the opening of the set screw bore and translated upwards along axis 45. The entrance of set screw bore 44 may have a diameter larger than the rest of bore 44. As for example, the diameter at the entrance of bore 44 may be slightly larger than the diameter of set screw lip 66, but the diameter of the rest of bore 44 may be slightly smaller than the set screw lip. This enables bore 44 to receive the set screw, but also prevents the set screw from inadvertently being removed or becoming displaced by sliding through the opposite outer end of set screw bore 44. Additionally, or in the alternative, set screw 20 may be provided with a radially extending groove 68 that is without threads. The external threads are disposed only on the part of the set screw above groove 68. Below groove 68, the set screw includes a smooth portion 69 or is at least without threads. Thus, even if the diameters of set screw 20 and bore 44 are similar throughout, smooth portion 69 is unable to cooperate with the threads of bore 44, thus prohibiting set screw 20 from becoming displaced from the outer end of bore 44. After set screw 20 is located within bore 44, ball ring 18 may then be placed within recessed portion 46, thus further locking the set screw within the bore. Set screw 20 may be prevented from backing out of bore 44.

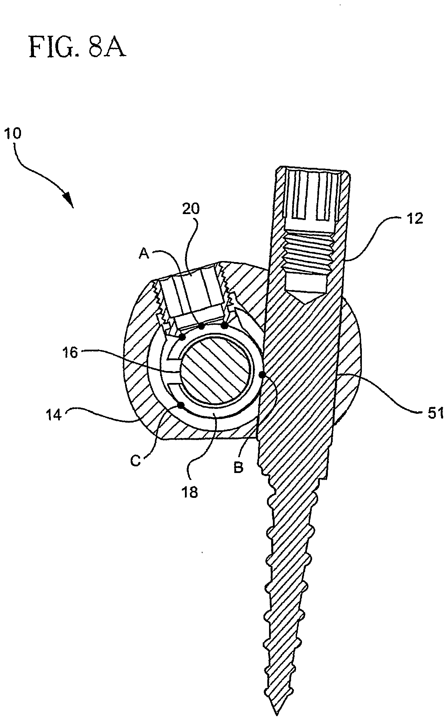

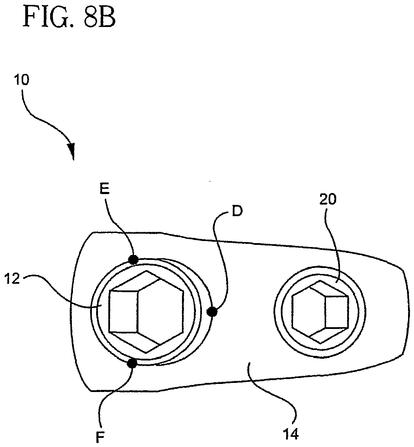

Compression of ball ring 18 occurs when set screw 20 is translated downward within set screw bore 44 until it begins to apply a pressure against ball ring 18. Prior to this, rod 16 is first placed within ball ring 18 and is slidably received by the opening 80. At this point, rod 16 may be angled within connector 14 with respect to the spinal column. Once aligned, ball ring 18 and rod 16, housed in the ball ring, are moved laterally by the force applied by the set screw. They continue to move laterally until ball ring 18 contacts stem 22 of bone fastener 12 located in first bore 40. By continuing translation of set screw 20 downward against ball ring 18, the ball ring, rod 16 and stem 22 of fixation element 12 move laterally until stem 22 abuts against an interior wall 51 of bore 40 of connector 14. At this point, stem 22 can no longer translate laterally, the continued pressure applied by the set screw against ball ring 18 causes ball ring 18 to compress and tighten around spinal rod 16 until a point is reached where ball ring 18 is fully tightened about spinal rod 16. Stem 22, spinal rod 16 and ball ring 18, which is part of the connector body, are now locked within the connector body relative to one another. The lateral movement of ball ring 18 and rod 16 is aided by the elliptical shape of channel 42. Additionally, the elliptical shape of channel 42 and bore 40 results in at least three points of contact for locking both ball ring 18 and stem 22, as shown in FIG. 3. First, ball ring 18, when in a locked position, may receive a force from or translates force to: (1) set screw 20; (2) stem 22; and (3) an interior wall 47 of channel 42 at positions A, B and C respectively in FIG. 8A. When in the locked position, stem 22 also may have at least three points of contact or three locations where it applies or receives pressure from: (4) ball ring 18; and (5) and (6) two points located on interior wall 51 of bore 40, D, E and F, respectively in FIG. 8B.

The use of ball ring 18 in the assembly also provides a greater friction surface area between not only set screw 20 and rod 16, but also the friction surface between stem 22 and ball ring 18 as compared with an assembly devoid of a ball ring. The cylindrical interface 84 of ball ring 18 has a radius about equal to that of rod 16, in such a way that ball ring 18 can slidingly receive rod 16. The spherical outer face 86 of ball ring 18 has a radius which is adapted such that, when positioned within connector 14, the walls or recessed portion 46 may contact outer face 86. The angular position of rod 16 engaged in ball ring 18 can be controlled in two mutually perpendicular planes over an amplitude of, for example, 15 degrees on either side of a mean position of the rod, in which the rod is perpendicular to the sagittal plane.

In the preferred method of operation of the bone fixation assembly, bone fixation element 12 is first engaged to a vertebral body 2 as shown in FIG. 1, preferably into a previously-drilled pilot hole in the bone. The bone fixation element is then screwed into the bone using a driver or other appropriate device, advancing the bone fixation element along its longitudinal axis into the vertebral bone. Bore 40 of connector 14 is aligned with stem 22 of bone fixation element 12, and the connector is moved in an anterior direction with first bore 40 receiving stem 22. At this point, connector 14 is still capable of being moved axially as well as rotationally about bone fixation element 12. Additionally, since ball ring 18 and set screw 20 are preassembled as previously described, ball ring 18 is located in channel 42 and ready to receive spinal rod 16, which is easily inserted through the ball ring opening 80. Prior to tightening the assembly, the angle of the fixation element is adjusted by moving connector 12 and the first bore 40 with respect to stem 21 of element 12. Due to the larger diameter of first bore 40 as compared to stem 22 of the bone fixation element, the bone fixation assembly can be manipulated to cover a broader range of angles for capturing an orthopedic stabilizing rod. Once bone fixation element 12, connector 14 and spinal rod 16 have all been positioned correctly, set screw 20 is translated downwardly through set screw bore 44 until the elements are secured relative to one another, as previously described. Achieving sufficient angulation between bone fixation elements while engaging the orthopedic rod is essential for assemblies mounted in spines having abnormal curvatures. Sufficient angulation is also important in the cervicothoracic junction of the spine. Thus, bone fixation assembly 10 can be locked at a wide range of angles relative to a vertebral body using a one-step process.

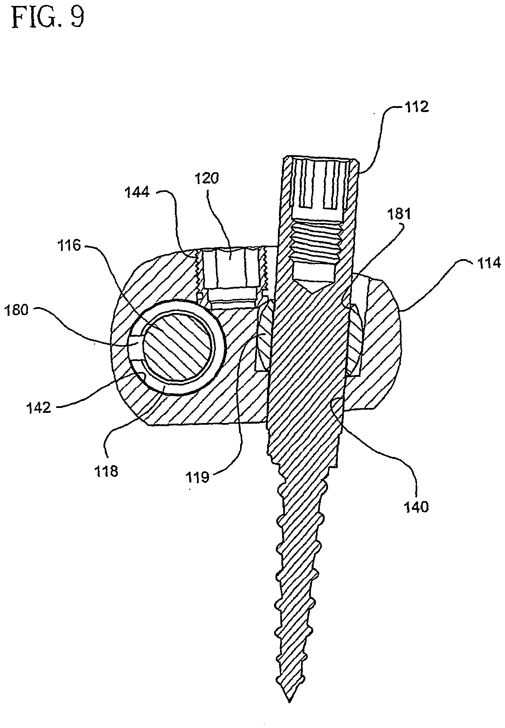

As shown in FIG. 9, in an alternate device which does not fall within the scope of the claims, set screw bore 144 may be positioned between first bore 140 and channel 142 with regard to connector 114. Set screw 120 is then translated downward within set screw bore 144, so as to come into contact with ball ring 118 as well as a second ball ring 119. Ball ring 118 works substantially the same as previously described. Specifically, set screw 120 applies a force against ball ring 118 constricting ball ring 118 so that it tightens around rod 116 disposed within the opening 180 of the ball ring. Additionally, as set screw 120 is translated downward into the set screw bore, the set screw simultaneously applies a pressure against second ball ring 119 located in first bore 140. A bone fixation element 112 is disposed in first bore 140 and has housed within its aperture 181 bone fixation element 114.

According to a preferred embodiment, bone fixation assembly 10 is supplied as a kit including a plurality of connectors 14 with ball rings 18 and set screws 20 pre-seated in each connector 14 to reduce the number of loose parts and prevent any small loose parts from being lost, or from having to be handled and manipulated during surgery. As used herein, the terminology "pre-seated" means that the elements are pre-assembled in a manner to then prevent from being inadvertently removed from their respective bores, as opposed to being loose in a package and requiring assembly of the individual components. However, the kit could include the set screws and rings as loose parts. The kit may also include a plurality of rods 16 and fixation elements 12. The size of the rods, as well as the size and type of fixation elements, may be varied within each kit and may also be different from kit to kit.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the scope of the present invention as defined by the appended claims.

### INDUSTRIAL APPLICABILITY

The present invention enjoys wide applicability including, but not limited to, medical devices such as orthopedic implants.

## Claims

1. A device (10) for securing a spinal rod to the spine comprising:
an elongated spinal rod configured to be implanted adjacent to the spinal column of a patient spanning across several vertebral bodies;
at least one fixation element (12) or engaging vertebral bodies at a number of vertebral levels, each of said fixation elements having a vertebral engaging portion (24) and a stem portion (22) extending from said vertebral engaging portion;
a ball ring (18) having a length and an aperture extending through the center of said ball ring, said ball ring further including a through slot that extends the entire lenght of said ball ring so as to be compressible and expandable about a central axis extending through the center of said ball ring, said aperture adapted to receive said spinal rod;
a set screw (20) having threads; and
a rod connector (14) for connecting said fixation element to said spinal rod, each of said rod connectors including a body defining a channel (42) adapted to house said ball ring, a stem bore (40) adapted to receive said stem portion therethrough, said stem bore in communication with said channel, and a set screw bore (44) including threads engageable with said threads of said set screw, wherein said set screw bore intersects said channel so as that when said set screw is threaded through said set screw bore, it is able to apply pressure to said ball ring, and said ball ring is able to apply pressure to said stem portion.

2. The device for securing a spinal rod to the spine according to claim 1, wherein said spinal rod is generally cylindrical.

3. The device for securing a spinal rod to the spine according to claim 1, wherein said stem portion of said fixation elements are smooth.

4. The device for securing a spinal rod to the spine according to claim 1, wherein said ball ring is captured within said channel of said connector.

5. The device for securing a spinal rod to the spine according to claim 1, wherein said through slot allows an outer diameter of said ball ring to be reduced less than a minimum diameter of said channel.

6. The device for securing a spinal rod to the spine according to claim 5, wherein said ball ring has a diameter smaller than a diameter of said channel of said rod connector so as to permit polyaxial motion of said rod when said rod is housed within said aperture of said ball ring.

7. The device for securing a spinal rod to the spine according to claim 1, wherein said set screw bore is located set off from said stem bore at an angle greater than or equal to 3 degrees with reference taken from said stem bore of said rod connector.

8. The device for securing a spinal rod to the spine according to claim 1, wherein said channel intersects said stem bore so as to permit said ball ring to apply pressure to said stem portion of said fixation element.

9. The device for securing a spinal rod to the spine according to claim 1, wherein said channel and said stem bore have a non-circular geometric configuration.

10. The device for securing a spinal rod to the spine according to claim 8, wherein when the device is in a locked position, the ball ring either applies pressure against or receives pressure from, at least three contact points, wherein said stem portion also either applies pressure against or receives pressure from, at least three contact points.

11. The device for securing a spinal rod to the spine according to claim 1, wherein said channel has an elliptical configuration, wherein said stem bore is offset at an angle between 3 degrees and 15 degrees relative to a second axis extending through the maximum distance of said channel.

12. The device for securing a spinal rod to the spine according to claim 11, wherein said set screw bore is aligned with said second axis.

13. The device for securing a spinal rod to the spine according to claim 1, wherein, said stem bore having a non-circular geometric shape, said non-circular geometric shape of said channel and said stem bore permit lateral movement of said ball ring and said stem portion when pressure is applied by said set screw against said ball ring and said shape also permits at least three contact points between said ball ring and an additional device or devices which either apply a force against said ball ring or receive a force from said ball ring and at least three contact points against said stem portion that either apply a force against said stem or receive a force from said stem.

14. The device for securing a spinal rod to the spine according to claim 13, wherein said stem portion is smooth.

15. The device for securing a spinal rod to the spine according to claim 13, wherein said set screw is captured in said connector.

16. The device for securing a spinal rod to the spine according to claim 13, wherein said stem bore is positioned at an angle between 3 and 15 degrees relative to a longitudinal axis passing through the center of said channel.

17. The device for securing a spinal rod to the spine according to claim 13, wherein said set screw bore is positioned at an angle greater than or equal to 5 degrees with reference taken from said stem bore of said connector.

## Patentansprüche

1. Vorrichtung (10) zur Fixierung einer Wirbelsäulenstange an der Wirbelsäule, die aufweist:
- eine längliche Wirbelsäulenstange, die konfiguriert ist, benachbart der Wirbelsäule eines Patienten implantiert zu werden, wobei sie mehrere Wirbelkörper überspannt;
- mindestens ein Fixationselement (12) zur Ineingriffnahme von Wirbelkörpern auf einer Anzahl von Wirbelhöhen, wobei jedes der Fixationselemente einen Wirbeleingriffsabschnitt (24) und einen Schaftabschnitt (22) aufweist, der sich vom Wirbeleingriffsabschnitt erstreckt;
- einen Kugelring (18), der eine Länge und eine Öffnung aufweist, die sich durch die Mitte des Kugelrings erstreckt, wobei der Kugelring ferner einen durchgehenden Schlitz aufweist, der sich über die gesamte Länge des Kugelrings erstreckt, so dass er um eine sich durch die Mitte des Kugelrings erstreckende Mittelachse zusammendrückbar und erweiterbar ist, wobei die Öffnung eingerichtet ist, die Wirbelsäulenstange aufzunehmen;
- einen Gewindestift (20) mit einem Gewinde; und
- ein Stangenverbinder (14) zur Verbindung des Fixationselements mit der Wirbelsäulenstange, wobei jeder der Stangenverbinder einen Körper, der einen Kanal (42) definiert, der eingerichtet ist, den Kugelring aufzunehmen, eine Schaftbohrung (40), die eingerichtet ist, den Schaftabschnitt dort hindurch aufzunehmen, wobei die Schaftbohrung mit dem Kanal in Verbindung steht, und eine Gewindestiftbohrung (44) aufweist, die ein Gewinde aufweist, das mit dem Gewinde des Gewindestifts in Eingriff bringbar sind, wobei die Gewindestiftbohrung den Kanal so kreuzt, dass wenn der Gewindestift durch die Gewindestiftbohrung geschraubt wird, er auf den Kugelring Druck ausüben kann und der Kugelring auf den Schaftabschnitt Druck ausüben kann.

2. Vorrichtung zur Fixierung einer Wirbelsäulenstange an der Wirbelsäule nach Anspruch 1, wobei die Wirbelsäulenstange im Wesentlichen zylindrisch ist.

3. Vorrichtung zur Fixierung einer Wirbelsäulenstange an der Wirbelsäule nach Anspruch 1, wobei der Schaftabschnitt der Fixationselemente glatt ist.

4. Vorrichtung zur Fixierung einer Wirbelsäulenstange an der Wirbelsäule nach Anspruch 1, wobei der Kugelring im Kanal des Verbindungsstücks aufgenommen ist.

5. Vorrichtung zur Fixierung einer Wirbelsäulenstange an der Wirbelsäule nach Anspruch 1, wobei es der durchgehende Schlitz ermöglicht, dass ein Außendurchmesser des Kugelrings auf weniger als einen Minimaldurchmesser des Kanals reduziert wird.

6. Vorrichtung zur Fixierung einer Wirbelsäulenstange an der Wirbelsäule nach Anspruch 5, wobei der Kugelring einen Durchmesser aufweist, der kleiner als ein Durchmesser des Kanals des Stangenverbinders ist, so dass eine mehrachsige Bewegung der Stange ermöglicht wird, wenn die Stange in der Öffnung des Kugelrings aufgenommen ist.

7. Vorrichtung zur Fixierung einer Wirbelsäulenstange an der Wirbelsäule nach Anspruch 1, wobei die Gewindestiftbohrung um einen Winkel von gleich oder größer 3 Grad bezüglich der Schaftbohrung des Stangenverbinders von der Schaftbohrung versetzt angeordnet ist.

8. Vorrichtung zur Fixierung einer Wirbelsäulenstange an der Wirbelsäule nach Anspruch 1, wobei der Kanal die Schaftbohrung kreuzt, um es zu ermöglichen, dass der Kugelring auf den Schaftabschnitt des Fixationselements Druck ausübt.

9. Vorrichtung zur Fixierung einer Wirbelsäulenstange an der Wirbelsäule nach Anspruch 1, wobei der Kanal und die Schaftbohrung eine nicht kreisförmige geometrische Konfiguration aufweisen.

10. Vorrichtung zur Fixierung einer Wirbelsäulenstange an der Wirbelsäule nach Anspruch 8, wobei, wenn sich die Vorrichtung in einer arretierten Position befindet, der Kugelring entweder Druck auf mindestens drei Kontaktpunkte ausübt oder Druck von ihnen aufnimmt, wobei auch der Schaftabschnitt entweder Druck auf mindestens drei Kontaktpunkte ausübt oder Druck von ihnen aufnimmt.

11. Vorrichtung zur Fixierung einer Wirbelsäulenstange an der Wirbelsäule nach Anspruch 1, wobei der Kanal eine elliptische Konfiguration aufweist, wobei die Schaftbohrung um einen Winkel zwischen 3 Grad und 15 Grad relativ zu einer zweiten Achse versetzt ist, die sich durch die maximale Strecke des Kanals erstreckt.

12. Vorrichtung zur Fixierung einer Wirbelsäulenstange an der Wirbelsäule nach Anspruch 11, wobei die Gewindestiftbohrung mit der zweiten Achse ausgerichtet ist.

13. Vorrichtung zur Fixierung einer Wirbelsäulenstange an der Wirbelsäule nach Anspruch 1, wobei der Kanal eine nicht kreisförmige geometrische Form aufweist, die Schaftbohrung eine nicht kreisförmige geometrische Form aufweist, die nicht kreisförmige geometrische Form des Kanals und der Schaftbohrung eine laterale Bewegung des Kugelrings und des Schaftabschnitts ermöglichen, wenn durch den Gewindestift auf den Kugelring Druck ausgeübt wird, und die Form au-βerdem mindestens drei Kontaktpunkte zwischen dem Kugelring und einer zusätzlichen Vorrichtung oder Vorrichtungen, die entweder eine Kraft auf den Kugelring ausüben oder eine Kraft vom Kugelring aufnehmen, und mindestens drei Kontaktpunkte am Schaftabschnitt ermöglicht, die entweder eine Kraft auf den Schaft ausüben oder eine Kraft vom Schaft aufnehmen.

14. Vorrichtung zur Fixierung einer Wirbelsäulenstange an der Wirbelsäule nach Anspruch 13, wobei der Schaftabschnitt glatt ist.

15. Vorrichtung zur Fixierung einer Wirbelsäulenstange an der Wirbelsäule nach Anspruch 13, wobei der Gewindestift im Verbinders aufgenommen ist.

16. Vorrichtung zur Fixierung einer Wirbelsäulenstange an der Wirbelsäule nach Anspruch 13, wobei die Schaftbohrung unter einem Winkel zwischen 3 Grad und 15 Grad relativ zu einer Längsachse angeordnet ist, die durch die Mitte des Kanals geht.

17. Vorrichtung zur Fixierung einer Wirbelsäulenstange an der Wirbelsäule nach Anspruch 13, wobei die Gewindestiftbohrung unter einem Winkel von gleich oder größer 5 Grad bezüglich der Schaftbohrung des Verbinders angeordnet ist.

## Revendications

1. Dispositif (10) pour fixer une tige vertébrale sur la colonne vertébrale, comprenant :
- une tige vertébrale allongée configurée pour être implantée de manière adjacente à la colonne vertébrale d'un patient couvrant plusieurs corps vertébraux ;
- au moins un élément de fixation (12) pour mettre en prise les corps vertébraux à un certain nombre de niveaux vertébraux, chacun desdits éléments de fixation ayant une partie de mise en prise vertébrale (24) et une partie de tige (22) s'étendant à partir de ladite partie de mise en prise vertébrale ;
- une bague sphérique (18) ayant une longueur et une ouverture s'étendant à travers le centre de ladite bague sphérique, ladite bague sphérique comprenant en outre une fente de passage sur toute la longueur de ladite bague sphérique afin d'être compressible et expansible autour d'un axe central s'étendant à travers le centre de ladite bague sphérique, ladite ouverture étant adaptée pour recevoir ladite tige vertébrale ;
- une vis de serrage (20) ayant des filetages ; et
- un connecteur de tige (14) pour raccorder ledit élément de fixation à ladite tige vertébrale, chacun desdits connecteurs de tige comprenant un corps définissant un canal (42) adapté pour loger ladite bague sphérique, un alésage de tige (40) adapté pour recevoir ladite partie de tige à travers ce dernier, ledit alésage de tige étant en communication avec ledit canal, et un alésage de vis de serrage (44) comprenant des filetages pouvant se mettre en prise avec lesdits filetages de ladite vis de serrage, dans lequel ledit alésage de vis de serrage coupe ledit canal de sorte que lorsque ladite vis de serrage est filetée dans ledit alésage de vis de serrage, elle peut appliquer une pression sur ladite bague sphérique, et ladite bague sphérique peut appliquer une pression sur ladite partie de tige.

2. Dispositif pour fixer une tige vertébrale sur la colonne vertébrale selon la revendication 1, dans lequel ladite tige vertébrale a une forme générale cylindrique.

3. Dispositif pour fixer une tige vertébrale sur la colonne vertébrale selon la revendication 1, dans lequel ladite partie de tige desdits éléments de fixation est lisse.

4. Dispositif pour fixer une tige vertébrale sur la colonne vertébrale selon la revendication 1, dans lequel ladite bague sphérique est capturée à l'intérieur dudit canal dudit connecteur.

5. Dispositif pour fixer une tige vertébrale sur la colonne vertébrale selon la revendication 1, dans lequel ladite fente de passage permet de réduire un diamètre externe de ladite bague sphérique selon une valeur inférieure à un diamètre minimum dudit canal.

6. Dispositif pour fixer une tige vertébrale sur la colonne vertébrale selon la revendication 5, dans lequel ladite bague sphérique a un diamètre inférieur à un diamètre dudit canal dudit connecteur de tige afin de permettre le mouvement polyaxial de ladite tige lorsque ladite tige est logée à l'intérieur de ladite ouverture de ladite bague sphérique.

7. Dispositif pour fixer une tige vertébrale sur la colonne vertébrale selon la revendication 1, dans lequel ledit alésage de vis de serrage est décalé dudit alésage de tige selon un angle supérieur ou égal à 3 degrés en référence audit alésage de tige dudit connecteur de tige.

8. Dispositif pour fixer une tige vertébrale sur la colonne vertébrale selon la revendication 1, dans lequel ledit canal coupe ledit alésage de tige afin de permettre à ladite bague sphérique d'appliquer une pression sur ladite partie de tige dudit élément de fixation.

9. Dispositif pour fixer une tige vertébrale sur la colonne vertébrale selon la revendication 1, dans lequel ledit canal et ledit alésage de tige ont une configuration géométrique non circulaire.

10. Dispositif pour fixer une tige vertébrale sur la colonne vertébrale selon la revendication 8, dans lequel lorsque le dispositif est dans une position bloquée, la bague sphérique applique une pression contre ou reçoit la pression d'au moins trois points de contact, dans lequel ladite partie de tige applique également une pression contre ou reçoit la pression d'au moins trois points de contact.

11. Dispositif pour fixer une tige vertébrale sur la colonne vertébrale selon la revendication 1, dans lequel ledit canal a une configuration elliptique, dans lequel ledit alésage de tige est décalé selon un angle compris entre 3 degrés et 15 degrés par rapport à un deuxième axe s'étendant sur la distance maximum dudit canal.

12. Dispositif pour fixer une tige vertébrale sur la colonne vertébrale selon la revendication 11, dans lequel ledit alésage de vis de serrage est aligné avec ledit deuxième axe.

13. Dispositif pour fixer une tige vertébrale sur la colonne vertébrale selon la revendication 1, dans lequel ledit canal ayant une forme géométrique non circulaire, ledit alésage de tige ayant une forme géométrique non circulaire, ladite forme géométrique non circulaire dudit canal et dudit alésage de tige permet le mouvement latéral de ladite bague sphérique et de ladite partie de tige lorsque la pression est appliquée par ladite vis de serrage contre ladite bague sphérique et ladite fente permet également au moins trois points de contact entre ladite bague sphérique et un dispositif ou des dispositifs supplémentaires qui appliquent une force contre ladite bague sphérique ou reçoivent une force de ladite bague sphérique, et au moins trois points de contact contre ladite partie de tige qui appliquent une force contre ladite tige ou reçoivent une force de ladite tige.

14. Dispositif pour fixer une tige vertébrale sur la colonne vertébrale selon la revendication 13, dans lequel ladite partie de tige est lisse.

15. Dispositif pour fixer une tige vertébrale sur la colonne vertébrale selon la revendication 13, dans lequel ladite vis de serrage est capturée dans ledit connecteur.

16. Dispositif pour fixer une tige vertébrale sur la colonne vertébrale selon la revendication 13, dans lequel ledit alésage de tige est positionné selon un angle compris entre 3 et 15 degrés par rapport à un axe longitudinal passant par le centre dudit canal.

17. Dispositif pour fixer une tige vertébrale sur la colonne vertébrale selon la revendication 13, dans lequel ledit alésage de vis de serrage est positionné selon un angle supérieur ou égal à 5 degrés en référence audit alésage de tige dudit connecteur.
